# EUROPEAN PATENT SPECIFICATION

(11) **EP 4 211 700 B1**
(45) Date of publication and mention of the grant of the patent: **30.10.2024**
(21) Application number: 21773049.8
(22) Date of filing: 02.09.2021
(51) Int. Cl.: G16H 40/67, A61B 3/00, G06F 21/00, A61B 3/14, G06F 21/31, G06F 21/62

(54) **MEDICAL DEVICE FOR AUTOMATIC EXAM ACQUISITION**
MEDIZINISCHE VORRICHTUNG ZUR AUTOMATISCHEN ERFASSUNG VON UNTERSUCHUNGEN
DISPOSITIF MÉDICAL POUR L'ACQUISITION AUTOMATIQUE D'EXAMEN

(30) Priority: 10.09.2020 DE 102020211406
(43) Date of publication of application: 19.07.2023
(73) Proprietor: IVIS TECHNOLOGIES S.r.l, 74100 Taranto (IT)
(72) Inventor: D IPPOLITO, Giuseppe, 74121 Taranto (IT)
(74) Representative: Bardehle Pagenberg Partnerschaft mbB Patentanwälte Rechtsanwälte
(86) International application number: PCT/EP2021/074251
(87) International publication number: WO 2022/053387

(56) References cited:
- WO-A1-2009/120543
- WO-A1-2014/080081
- WO-A1-2020/203323
- JP-A- 2004 275 591
- US-A1- 2016 183 796
- US-A1- 2018 220 889
- US-A1- 2020 093 361

## Description

### 1. Technical field

The present invention relates to medical devices.

### 2. Background

Medical devices play an important role in modern medicine, and as technology advances, their importance is only set to grow. Medical devices find application in virtually every single branch of medicine, facilitating and enhancing diagnosis and treatment of countless diseases and conditions.

In current practice, medical devices need to be operated by trained personnel, in particular medical personnel such as physicians, technicians and/or nurses. Such trained personnel does not only have to identify, e.g., which exam of what target structure of a patient is to be acquired. Trained personnel is, *inter alia,* also required to then correctly position the target structure with respect to the medical device. Last but not least, trained personnel is also required to operate and supervise the medical device during exam acquisition.

There is therefore a need to reduce the requirements as to training of operators and/or the extent to which their intervention/assistance is required.

### 3. Summary of the invention

This need is met, at least in part, by the present invention.

In a first aspect, the present invention relates to a medical device for acquiring an exam of a patient for medical purposes that comprises means for automatically acquiring the exam.

Such a medical device allows for an exam to be automatically acquired, such that no trained personnel is required to prepare, perform and/or evaluate exam acquisition at all. To the contrary, the entire process may be handled by the patient him- or herself, allowing to avoid the presence of another individual in physical proximity to the patient altogether. For example, the device may comprise a start button to be pressed by a patient, upon which the device acquires an exam of the patient, preferably acquiring unique features of an examined structure of the patient for automatic exam identification in a fully automatized way. In another example, not even a start button needs to be pressed. Instead, the device automatically recognizes the patient upon which the acquisition of the exam may start.

Thereby, the present invention may provide at least the following three advantages:
- It may render exam acquisition less time-consuming and expensive.
   Conventionally, trained personnel is directly involved in receiving the patient, inputting the patient's personal data, positioning the patient's structure to be examined relative to the medical device, as well as in executing and evaluating the exam as such. These activities are time-consuming and therefore represent considerable costs for the institutions for which said trained personnel works, e.g., hospitals or private clinics.
   The present invention dispenses with the need to involve trained personnel in these activities, thereby reducing the overall cost of operating the medical device.
- It may render exam results operator-independent.
   In conventional approaches, it is the operator of the medical device, i.e., usually trained personnel, who identifies the target structure and positions it with respect to the medical device. Thus, exam results are, at least to a certain degree, dependent on the operator, e.g., on his or her experience. This may lead to unpredictable and/or inaccurate exam results.
   In contrast, the present invention allows to perform exam acquisition automatically, i.e., without any manual input from trained personnel that could induce variations from exam to exam, rendering the process and also exam results perfectly objective.
- It may decrease the risk of infection.
   Conventionally, the operator of the medical device and the patient are generally close to each other during preparation and acquisition of the exam. But physical proximity, especially in closed environments, is a well-known catalyst for transmission of infections. Hence, exam acquisition using medical devices as known in the art increases the risk of (mutual) infection for both the operator as well as the patient.
   The present invention allows that patient and operator do not need to be in physical proximity to each other at any time, thereby significantly reducing the risk of (mutual) infection for both the operator and the patient.

A medical device according to the present invention may be any medical device, for any medical and/or clinical purpose, and/or for acquiring an exam of any target structure of a patient. For example, the medical device may be for acquiring an exam of an eye of a patient, of a throat, an ear or a nose of a patient, of a limb of a patient, of a torso of a patient (front and/or back). The medical device may be an imaging device, such as a magnetic resonance imaging scanner or nuclear magnetic resonance imaging scanner, a positron emission tomographer, a computer tomographer, an x-ray device, a laser-based device, a light scattering-based device for tomography, a light reflection-based device for topography, a device to analyze the pupil, a refractometer device, a device based on optical coherence tomography and/or an ultrasound-based device.

In particular, a medical device according to the present invention may not be a wearable device, e.g., it may be a device that is not wearable and/or portable with one hand. Rather, the medical device may be, e.g., a stationary device. The patient may place himself/herself in front of the device. Upon automatic recognition of the patient by the device and/or pressing a start button of the device, the exam may be automatically performed. The term "stationary" as understood herein is to include devices that are transportable, e.g. that can be rolled on wheels.

For example, the medical device may be an ophthalmic medical device. In some examples, the medical device may be a tomographer, e.g., a corneal tomographer. For example, the tomographer may be adapted to provide profiles of the anterior and/or posterior surface of the cornea and/or intermediate interfaces of the different layers of the cornea and a thickness map of the different layers of the cornea of an eye of a patient. The patient may place himself/herself in front of the device. For example, the patient may stand in front of the device, possibly placing his/her chin on a chin rest. Upon automatic recognition of the patient by the device and/or pressing a start button of the device, an exam recording data of the cornea of the eye(s) to be examined is automatically performed.

In an example, the medical device may comprise means for switching the medical device into a first mode dedicated for automatically acquiring an exam for medical purposes. Next to said first mode, there may also be at least one second mode in which at least one functionality of the medical device that is disabled in the first mode is enabled. In an example, the medical device may also comprise means for switching the medical device into said at least one second mode. That is, when the medical device is in the first mode dedicated for automatically acquiring an exam, at least one functionality of the medical device may be disabled. For example, the only functionality offered for control by the medical device in the first mode may be to start and/or stop exam acquisition. In some examples, trained personnel may switch the medical device into the first mode, e.g., at a preselected date and time, for example based on an appointment of the patient. Such a mode of reduced functionalities minimizes the risk of failure of automatic exam acquisition as it may avoid any wrong or unnecessary actions of the patient which may affect the exam results. Of course, such a mode thereby also increases safety.

In some instances, it may be possible to retroactively provide an already existing medical device with means for switching the medical device into a first mode dedicated for automatically acquiring an exam for medical purpose. This may allow to provide already existing medical devices with the capability of automatically acquiring an exam. For example, the means for switching may comprise a module and/or a processor that may be interfaced with an already existing medical device in order to provide it with the capability of automatically acquiring an exam.

In an example, the medical device may comprise means for determining whether the patient is authorized to use the medical device for automatically acquiring the exam. In some examples, an exam may only be automatically acquired if (and only if) the patient is authorized to use the medical device for automatically acquiring the exam. Conversely, in some examples, automatic exam acquisition and/or the medical device may be shut down if it cannot be determined whether the patient is authorized, e.g. because the patient is not authorized. Additionally or alternatively, trained personnel may be informed that it cannot be determined whether the patient is authorized. In some cases, this may be, e.g., because the patient is in fact not authorized. In other cases, this may be, e.g., because an error occurred during the determining. In this manner, it may be ensured that automatic exam acquisition is indeed only performed if the patient is authorized, e.g. is a suitable candidate for automatic exam acquisition, is well familiar with the medical device, and/or knows how to use the medical device, thereby reducing the risk that the patient takes any wrong or unnecessary actions during automatic exam acquisition, thereby in effect increasing accuracy and safety of automatic exam acquisition with the medical device.

In some examples, the means for determining whether the patient is authorized to use the medical device for automatically acquiring the exam may be adapted to determine whether the patient is authorized based at least in part on a code, such as a barcode or a quick response, QR, code. That is, whether the patient is authorized to use the medical device for automatically acquiring the exam may be conveyed by and/or embedded in such a code. For example, such a code may only be issued to the patient upon completion of a training and/or a test as will be described below. Moreover, the patient's personal data and/or preferred language may also be embedded in the code; and, optionally, the code may only be issued to the patient if the patient has provided his or her personal data and/or preferred language. Similarly, information on the target structure to be examined may be embedded in the code; and, optionally, the code may only be issued to the patient if a target structure has been specified. Correspondingly, the medical device may further comprise means for acquiring the code, such as a camera or a scanner, e.g. a barcode or QR code scanner. This provides a particularly user-friendly way for the patient to demonstrate his or her authorization and/or provide his or her personal data and/or preferred language to the medical device.

For example, the patient may only be authorized to use the medical device for automatically acquiring the exam after he or she at least partly completed a training and/or a test, e.g., a multiple-choice test, regarding the medical device. In some examples, the patient may be required to first complete the training before proceeding to the test. In some examples, if the patient fails the training and/or test at least partly, he/or she may be asked to contact trained personnel, either for, e.g., personal instruction or for exam acquisition by the trained personnel instead of automatic exam acquisition.

The training may for example be at least partly audio-based and/or video-based. For example, the patient may be shown a video demonstrating how the medical device may be used for automatic exam acquisition. Additionally or alternatively, the training may be augmented reality-based and/or virtual reality-based. That is, using suitable equipment, the patient may interact with a model of the medical device, providing a particularly immersive and hence effective way for the patient to learn how to use the medical device, in particular for automatic exam acquisition. Both the training and/or the test may be based at least partly on a step-by-step model of exam acquisition. In some examples, the patient may be required to correctly answer a question regarding a given step before he or she is allowed to proceed to the next step within the training and/or test. This ensures that the patient understands each and every step that is to be taken during automatic exam acquisition, further enhancing accuracy and safety of automatic exam acquisition with the medical device.

In some examples, in order to be authorized to use the medical device for automatically acquiring an exam, the patient may also need or be requested to provide his or her personal data. This may allow acceleration of the patient's exam, as the medical device may then readily acquire necessary personal data regarding the patient when determining whether he or she is authorized to use the medical device for automatically acquiring the exam. Also, providing personal data as part of the authorization may facilitate privacy, e.g., safeguarding the patient's personal data in compliance with all relevant international standards.

Similarly, in some examples, in order to be authorized to use the medical device for automatically acquiring an exam, the patient may also need or be requested to indicate a preferred language. The medical device may then automatically use and/or switch to the patient's preferred language to be used for interacting with the patient, e.g., by means of a user interface as will be described in more detail below. This may accelerate automatic exam acquisition.

The means for determining whether the patient is authorized may comprise means for determining a target structure to be examined. That is, in some examples, the patient may not be authorized to use the medical device for automatically acquiring any exam with the medical device. Rather, the patient may only be authorized to use the medical device for automatically acquiring an exam of a specific target structure. Possibly, trained personnel, e.g., a physician or a nurse, may inform the patient of which target structure is to be examined. Exemplarily, considering a human eye as target structure, an exam may be required for the right eye (oculus dexter, OD), left eye (oculus sinister, OS) or both (oculus uterque, OU). In some examples, a training and/or a test the patient may need to complete as described above may be accordingly limited. That is, the training and/or test may specifically teach and test use of the medical device for automatically acquiring an exam of the specific target structure, thereby further enhancing accuracy and safety of automatic exam acquisition of the specific target structure with the medical device. Moreover, the medical device will then only perform the authorized exam, such that performing superfluous exam acquisitions may be avoided.

In some examples, the patient may be authorized to use the medical device for automatic exam acquisition by a corresponding computer program. In particular, such a computer program may comprise a web-based application and/or a smartphone and/or tablet application, providing a particularly user-friendly way to obtain authorization. Using a computer program to authorize the patient, it may be particularly easy and user-friendly to implement the above-mentioned training and/or test, e.g., in the shape of a guided wizard procedure. Also, a computer-program may be well-suited to issue an authorization, e.g. a barcode or QR code as described above, conditional on the patient being authorized. For example, the patient may complete a training and/or test on his or her smartphone (application) and then be issued a code as described above. For instance, the code may be displayed on the display of his or her smartphone or stored thereon for later (repeat) display. If the patient then seeks to automatically acquire the exam, he or she may simply use his or her smartphone to present the code to means for acquiring the code of the medical device.

A medical device according to the present invention may comprise means for identifying and/or tracking a target structure of the patient. For example, such means may comprise a camera. Furthermore, such means may comprise one or more algorithms for matching the target structure and a reference structure. The means for identifying and/or tracking may consider all six degrees of freedom in three-dimensional space. Means for identifying and/or tracking may ensure that the correct target structure is being examined, thereby enhancing safety and accuracy of automatic exam acquisition. For example, the means for identifying and/or tracking may identify the target structure at the very beginning of the automatic exam acquisition procedure. In some examples, automatic exam acquisition may start upon (successful) identification of the target structure, without requiring any further input from the patient. Conversely, in some examples, automatic exam acquisition and/or the medical device may be shut down and/or the patient may be requested to contact the operator of the medical device and/or accordingly trained personnel if the means for identifying and/or tracking fail to identify the target structure. Similarly, the means for identifying and/or tracking may track whether the target structure remains correctly positioned with respect to the medical device, e.g. during exam acquisition, in turn further enhancing safety and efficacy of automatic exam acquisition. Conversely, in some examples, automatic exam acquisition and/or the medical device may be aborted and/or shut down and/or the patient may be requested to contact the operator of the medical device and/or accordingly trained personnel if the means for identifying lose track of the target structure.

A medical device according to the present invention may in some examples also comprise means for adjusting a position of a target structure of the patient relative to at least a portion of the medical device, for example using at least one motor. This may allow automatizing initial positioning of the patient and/or the target structure relative to the medical device or at least the relevant portion thereof that is to interact with the target structure (e.g., an aperture of an imaging system, a lens of a camera system, etc. in case of a medical imaging device), respectively. Similarly, means for adjusting a position of a target structure of the patient relative to at least a portion of the medical device may additionally or alternatively also allow to automatically keep the patient and/or the target structure in the correct position with respect to the medical device throughout automatic exam acquisition or at least parts thereof. Notably, means for adjusting as just described may be adapted to adjust the position of the target structure of the patient relative to the medical device either by moving the patient and/or the target structure, or by moving the medical device or at least the relevant portion of the medical device (such as, e.g., a lens in case of a medical imaging device), or both. Automatically adjusting the position of the target structure of the patient relative to at least a portion of the medical device - whether initially or continually or both - may enhance safety and accuracy of automatic exam acquisition since it ensures that the target structure is and/or remains in a position suitable for automatic exam acquisition relative to the medical device or at least its relevant portion (such as, e.g., a lens in case of a medical imaging device), respectively.

In some examples, the means for adjusting may be adapted to adjust the position of the target structure based at least in part on feedback provided from the means for identifying and/or tracking, e.g. based on a current position of the target structure determined by the means for identifying and/or tracking. That is, the means for adjusting and means for identifying and/or tracking as described above may interact, providing for particularly safe and reliable initial and/or continued positioning of the target structure relative to the medical device.

In some examples, the medical device may comprise a user interface, e.g., a screen, a touchscreen and/or an audio output. Such user interface may be adapted to provide feedback and/or orders to the patient. For example, the user interface may guide the patient through the entire automatic exam acquisition process, or only through one or more parts thereof. For example, the user interface may provide means for the patient to start and/or stop automatic exam acquisition, e.g., in the form of corresponding buttons. The user interface may for example also instruct the patient how to position him-/herself relative to the medical device. Also, it may provide feedback, e.g. affirmative feedback upon successful positioning and/or upon any other steps successfully completed by the patient. The user interface may also be used to inform the patient of the result(s) of automatic exam acquisition. All in all, a user interface enables particularly user-friendly automatic exam acquisition, thereby reducing the risk that the patient may take any unnecessary or incorrect actions, in turn rendering automatic exam acquisition safer and more accurate. Also, it may improve patient experience and may make patients feel safer.

In some examples, the medical device may comprise means for validating automatic exam acquisition based on comparing a plurality of results obtained from acquiring the exam of the patient a plurality of times. For example, for validating automatic exam acquisition, it may be ascertained whether any differences between two or more results of the plurality of results are within predefined margins and/or standards. In some examples, whether automatic exam acquisition is validated or not may be shown to the patient by means of, e.g., a pass-fail message, possibly also a pass-with-reservation message, e.g., using a user interface as described above. In some examples, such a pass-fail message may comprise intuitive color coding, such as green (pass), yellow (pass with reservation) or red (fail), or intuitive imagery such as corresponding emojis and/or smileys. If the user interface comprises an audio output, e.g. one or more speakers, also a voice message may be released, stating the pass or fail result. In some examples, the patient may be requested to perform automatic exam acquisition once again if the previous attempt cannot or is not validated. Alternatively, or if automatic exam acquisition is not validated for a predefined number of times, automatic exam acquisition and/or the medical device may shut down and/or the patient may be requested to contact the operator and/or accordingly trained personnel.

In a second aspect, the present invention relates to a computer program for authorizing a patient to use a medical device to automatically acquire an exam of the patient for medical purposes. Specifically, such a computer program comprises code for determining one or more criteria based at least in part on one or more properties of the medical device as well as code for verifying whether the patient meets at least one of the criteria. For example, one of these criteria may be whether a training and/or a test regarding the medical device as described above has been completed. As already mentioned above, such a computer program - especially when provided in the form of a web-based application and/or a smartphone and/or tablet application - provides a particularly user-friendly way for the patient to be authorized.

In a third aspect, the present invention relates to a computer-processable authorization of a patient to use a medical device to automatically acquire an exam of the patient for medical purposes. In some examples, such an authorization may comprise a code, such as a barcode or a quick response, QR, code as already described above. In some examples, such an authorization may comprise at least one of personal data concerning the patient, information concerning the exam of the patient and/or information concerning a target structure of the patient. In some examples, such an authorization may be provided by a computer program as described above, enabling seamless integration and hence increasing user-friendliness. A computer-processable authorization may provide a particularly failsafe and efficient way for the patient to demonstrate to the medical device that he or she is authorized to use the medical device to automatically acquire an exam.

In a fourth aspect, the present invention relates to a system for acquiring an exam of a patient for medical purposes, comprising a medical device as described above as well as at least one of a computer program as described above and/or a computer-processable authorization as described above.

### 4. Brief description of the Figures

Possible examples of the present invention will be described in more detail in the subsequent detailed description with reference to the following figures:
- Fig. 1:: Exemplary medical device for acquiring an exam of a patient for medical purposes according to some aspects of the present invention.
- Figs. 2A, 2B:: Flowchart illustrating operation of an exemplary medical device for acquiring an exam of a patient for medical purposes according to some aspects of the present invention.

### 5. Detailed description of possible examples

For the sake of brevity only a few examples will be described in the following. The skilled person will recognize that the specific features described with reference to these examples may be modified and combined differently and that individual features may also be omitted if they are not essential. The general explanations in the sections above are also valid for the following more detailed explanations.

A medical device for acquiring an exam of a patient for medical purposes according to an aspect of the present invention may be used in conjunction with a computer program for authorizing the patient to use the medical device to automatically acquire the exam of the patient for medical purposes according to another aspect of the present invention, for example as described in the following.

Patients who are potential candidates for automatic exam-acquisition according to an aspect of the present invention may preferably, but not mandatorily, be identified by trained personnel such as a physician, a technician or a nurse. Trained personnel may also identify a target structure in or of the patient to be examined. For example, considering the human visual system, i.e., eyes, as a target structure, trained personnel (e.g., an operator of a suitable medical device) may inform the patient about the eye to be acquired: right eye, left eye or both.

Before the patient may then use a suitable medical device to automatically acquire an exam of the target structure, he or she may be required to use, e.g., a dedicated patient-interactive application, i.e., a computer program for authorizing the patient to use the medical device to automatically acquire the exam of the patient for medical purposes according to an aspect of the present invention, to be trained and/or taught about the medical device, e.g., how to use the medical device, for instance, for automatic exam-acquisition. Additionally or alternatively, the dedicated patient-interactive application may be used to authorize the patient to use the medical device to automatically acquire the exam. For example, the dedicated patient-interactive application may authorize, e.g., confirm, that the patient may use the medical device to automatically acquire the exam. In an example, the dedicated patient-interactive application may issue a computer-processable authorization of a patient to use a medical device to automatically acquire an exam of the patient for medical purposes according to another aspect of the invention. Preferably, but not mandatorily, the dedicated patient-interactive application may be downloaded by the patient by means of a dedicated web-link, possibly including operating instructions and/or a manual. In some examples, the dedicated patient-interactive application - hereinafter also occasionally dubbed Device Educative Interactive Tutorial (DEIT) - may be used, e.g., installed and/or executed, in a web browser (web application) or on a touch-screen mobile device (smartphone or tablet) or on a standard PC.

DEIT may preferably, but not mandatorily, comprise two separate sections, the first one to train and/or teach the patient to use the medical device to automatically acquire an exam and the second one to authorize the patient to use the medical device to automatically acquire an exam. That is, the second section may be used to authorize, e.g., confirm, that the patient is trained, taught and/or allowed to perform automatic exam acquisition using the medical device.

Preferably, but not mandatorily, the first section may require the patient to look, e.g., at a tutorial reproducing all steps needed to perform automatic exam acquisition using the medical device. The tutorial may comprise one or more videos and/or audio instructions and/or a guided wizard procedure.

After following and/or completing the tutorial, the patient may be preferably, but not mandatorily, be required to enter the second section of DEIT in order to be authorized to use the medical device to automatically acquire an exam. The second section may preferably, but not mandatorily, concern, e.g., reproduce or model, automatic exam acquisition using the medical device step by step.

Preferably, but not mandatorily, for each of these steps, the patient may be required to select an action to be taken, e.g., from a list of possible actions (multiple choice). Preferably, but not mandatorily, the second section of DEIT may only move to a next step if the patient selects the correct action for a current step. Preferably, but not mandatorily, if the patient fails to pass one or more steps, the patient may be requested to contact an operator of the medical device to undergo an operator-driven exam. Conversely, if the patient passes all steps, i.e., the entire second section of DEIT, the patient may preferably, but not mandatorily, be requested to enter his personal data and the preferred spoken language.

Preferably, but not mandatorily, if the patient passes all steps, i.e., the entire second section of DEIT, a unique code, such as a barcode and/or QR code or any other suitable code, may be generated and displayed and/or stored. Optionally, the patient may also be informed of a place, date and time to perform automatic exam acquisition. Alternatively, he or she may be requested to contact the operator of the medical device to set a place, date and time to perform to perform automatic exam acquisition. Optionally, e.g., upon closing DEIT, the patient may preferably, but not mandatorily, be asked to bring the unique code with him.

Preferably, a medical device for acquiring an exam of a patient for medical purposes according to an aspect of the present invention may be switched into a first mode dedicated for automatically acquiring an exam for medical purposes, hereinafter also occasionally dubbed Patient Dedicated Configuration (PDC). In PDC, the medical device may be configured to identify an authorized patient. Furthermore, in PDC, the medical device may be configured to support the patient during automatic exam acquisition. Preferably, but not mandatorily, this may comprise limiting the interaction between the patient and the medical device, e.g., the patient may only be able and/or allowed to perform actions to start and/or stop exam acquisition.

Preferably, but not mandatorily, the medical device may be switched into PDC by an operator of the medical device, e.g., trained personnel such as a physician or a nurse. In some examples, the operator may be physically present during exam acquisition, but in other examples he or she may not be. However, preferably, the operator should be nearby to intervene in case of need.

In an example, when the patient arrives at a preselected date and time to automatically acquire an exam using the medical device, the medical device may then already be in PDC and provide only a start button using a user interface including, e.g. a display or screen, optionally with touch functionality, as well as one or more speakers. Additionally, the medical device may release, at predefined time intervals, a voice message requesting the patient to press the start button in order to initiate automatic exam acquisition.

After pressing the start button, the patient may preferably, but not mandatorily, be requested to present the unique code mentioned above to the medical device, e.g., to means for acquiring the code. For example, the patient may be requested to hold and/or move the unique code in front of a dedicated scanning system, such as a camera and/or a code scanner or any other suitable device. Preferably, but not mandatorily, a corresponding image or video may be shown via the user interface of the medical device in order to support the patient in holding the unique code in the right position and/or orientation for scanning. In other examples, the exam may be started by presenting the code without the need to press a start button. Preferably, but not mandatorily, if the code may not be successfully scanned within a predefined lapsing time, automatic exam acquisition and/or the medical device may shut down. Additionally or alternatively, the patient may be required to contact the operator of the medical device. Conversely, if the code may be successfully scanned within the predefined lapsing time, the medical device may list, using its user interface, all necessary patient and exam related data, such as the patient's identity, his or her electronic medical record, his or her spoken and/or preferred language, target structure to be examined and/or any other data that may be embedded and/or specified in the unique code.

In some examples, the patient may then be required to confirm the data, e.g., by means of a confirmation button provided by the user interface of the medical device. Possibly, the data may then be automatically stored in a database of the medical device or any other suitable location. If data regarding the patient is already stored, said data may be updated accordingly.

Subsequently, the patient may preferably, but not mandatorily, be requested to proceed with automatic exam acquisition. For example, a voice message, released at predefined time intervals, may ask the patient to press a corresponding button provided by the user interface. If the patient does not (wish to) proceed with automatic exam acquisition, e.g., if the button is not pressed within a predefined lapsing time, automatic exam acquisition and/or the medical device may shut down. Additionally or alternatively, the patient may be required to contact the operator of the medical device.

At any time, one or more voice messages, released at predefined time intervals, may guide the patient during automatic exam acquisition.

The patient may be asked and/or required to perform exam acquisition a plurality of times, such that automatic exam acquisition may be validated, e.g., based on comparing a plurality of results obtained from acquiring the exam the plurality of times. If the exams are not acquired the plurality of times within a predefined lapsing time, automatic exam acquisition and/or the medical device may shut down. Additionally or alternatively, the patient may be required to contact the operator of the medical device. Conversely, if the exams are acquired the plurality of times within the predefined lapsing time, the patient may be asked to wait until exam validation is completed.

In some examples, exam validation may comprise checking that differences between a plurality of results obtained from acquiring the exam the plurality of times are within predefined margins and/or standards. In some examples, whether automatic exam acquisition is validated or not may be shown to the patient by means of, e.g., a pass-fail message as already described above.

Preferably, if exam validation is successful, a result of automatic exam acquisition may be stored. Additionally or alternatively, the result may be presented to the patient using the user interface of the medical device, e.g., shown to the patient on a display or screen of the medical device, and/or released in the form of a voice message, possibly at predefined time intervals.

If exam validation is only partly successful (pass with reservation), the patient may be allowed to restart automatic exam acquisition, i.e., to perform automatic exam acquisition once again. If exam validation is only partly successful for a predefined number of times, automatic exam acquisition and/or the medical device may shut down. Additionally or alternatively, the patient may be requested to contact the operator and/or accordingly trained personnel. Optionally, a result of automatic exam acquisition may be stored.

Preferably, but not mandatorily, the patient may eventually be informed that automatic exam acquisition is complete. Additionally or alternatively, the patient may be asked to contact the operator and/or accordingly trained personnel to evaluate and/or discuss the result of automatic exam acquisition.

Preferably, a medical device according to an aspect of the present invention may comprise means for identifying and/or tracking a target structure of the patient. For example, a suitable pattern matching (PM) application may be implemented in the medical device to identify the target structure to be examined. In some examples, the PM application may also allow tracking the movements of the target structure during exam acquisition. The PM application may consider up to all six degrees of freedom in three-dimensional space.

The PM application may identify (e.g., recognize) the target structure, preferably, but not mandatorily, by means of fitting algorithms that compare the target structure as detected by the medical device with reference target structures. Such reference target structures may have been detected by the medical device during a previous exam acquisition, imported, e.g., by means of different media, or stored beforehand.

Preferably, the PM application may seek to identify the target structure as soon as information regarding the target structure is provided to the medical device. In other examples, the PM application may seek to identify the target structure upon a button being pressed. If the PM application fails to identify the target structure within a predefined lapsing time, automatic exam acquisition and/or the medical device may shut down. Additionally or alternatively, the patient may be required to contact the operator of the medical device.

Upon identification of the target structure, the PM application may preferably, but not mandatorily, generate data specifying a position and/or orientation of the target structure in three-dimensional space.

In some examples, the PM application may also perform the above continuously, thereby tracking the target structure, e.g. during exam acquisition. That is, the PM application may continuously generate or update data specifying a position and/or orientation of the target structure in three-dimensional space.

Preferably, a medical device according to an aspect of the present invention may also comprise means for adjusting a position of a target structure of the patient relative to at least a portion of the medical device. In particular, the means for adjusting a position - hereinafter also occasionally dubbed motorized sub-system - may advantageously interact with the PM application, i.e., with the means for identifying and/or tracking a target structure of the patient. For example, upon identification of the target structure and generation of corresponding data specifying a position and/or orientation of the target structure in three-dimensional space, the motorized sub-system may move the target structure into a region of interest (ROI) of the medical device. The ROI may for example be a region in which a target structure needs to be placed for the medical device to acquire an exam thereof. In some embodiments, exam acquisition starts automatically once the target structure is moved to or positioned in the ROI of the medical device. Optionally, the user interface of the medical device informs the patient thereof.

Similarly, the motorized sub-system may keep the target structure within the ROI of the medical device, continuously moving it back into the ROI of the medical device (e.g., by re-positioning and re-orientating the target structure) upon an undesired or unexpected movement of the target structure relative to the medical device.

In some examples, if the motorized sub-system fails to keep and/or return the target structure within/to the ROI of the medical device within a predefined lapsing time, automatic exam acquisition and/or the medical device may shut down. Additionally or alternatively, the patient may be required to contact the operator of the medical device.

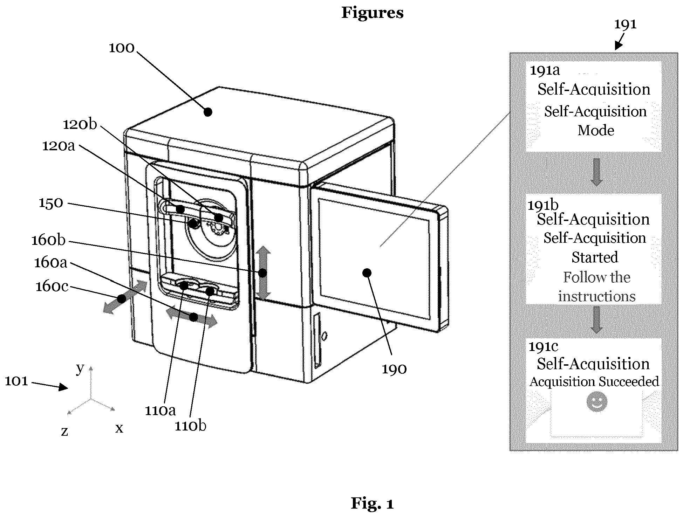

An exemplary medical device 100 comprising means for automatically acquiring an exam according to the present invention is shown in Fig. 1. Medical device 100, in the example shown in Fig. 1, is an ophthalmic medical device. That is, medical device 100 is adapted to automatically acquire an exam of an eye of a patient (not shown), e.g., by means of a main camera 150 that may be adapted to rotate around the eye of the patient, e.g., if the eye (e.g. the eye's optical axis) is aligned with an axis around which main camera 150 is adapted to rotate. Main camera 150 may be adapted to acquire a sectional side view of the eye, in particular of the cornea of the eye. Medical device 100 may be a stationary device.

Medical device 100 may comprise means for determining whether the patient is authorized to use medical device 100 for automatically acquiring the exam as described above. For example, the means for determining whether the patient is authorized may be adapted to determine whether the patient is authorized based at least in part on a code, preferably a barcode or a QR code. To this end, medical device 100 may further comprise means for acquiring such a code. For medical device 100, such a code may also store information on which eye (left, right or both) of the patient is to be examined. Moreover, such a code may store various other information, e.g., regarding the patient's identity and/or medial record, as described in detail above.

Medical device 100 may comprise a chin rest and/or a forehead rest. In the example of Fig. 1, chin rests 110a and 110b as well as forehead rests 120a and 120b are provided. If an exam is to be acquired of the patient's right eye, the patient may need to and/or be requested to place his chin on chin rest 110a and his forehead against forehead rest 120a. Chin rest 110a and forehead rest 120a may be arranged left to a center of the medical device 100. If an exam is to be acquired of the patient's left eye, the patient may need and/or be requested to place his chin on chin rest 110b and his forehead against forehead rest 120b. Chin rest 110b and forehead rest 120b may be arranged right to a center of the medical device 100. Medical device 100 may detect whether the patient placed his chin on chin rest 110a or 110b by means of one or more infrared sensors comprised in chin rest 110a, chin rest 110b or both (and/or in forehead rest 120a, forehead rest 120b, or both).

Medical device 100 comprises means for identifying and/or tracking a target structure of the patient as described above. That is, medical device 100 comprises means for identifying and/or tracking the eye of the patient to be examined. For example, main camera 150, which is adapted to rotate around the eye of the patient to be examined, may be used to determine the position of the eye to be examined in z -direction (cf. coordinate system 101 in Fig. 1). An auxiliary camera may be used to determine the position of the eye to be examined in x- and y-directions (cf. coordinate system 101).

The auxiliary camera may be adapted to acquire a front view of the eye. For example, the position of the pupil center (x-y) and/or the corneal apex (z) may be identified and tracked by adjusting a position of the eye of the patient (described further below). Medical device 100 and/or the means for identifying and/or tracking, respectively, may furthermore comprise means for determining an orientation of the eye of the patient. Determining an orientation may be done at least partly based on computations. Such computations may comprise one or more image transformations. That is, medical device 100 may comprise means for determining both a position as well as an orientation of the eye of the patient in three-dimensional space. In other words, the means for identifying and/or tracking of medical device 100 may take into account six degrees of freedom.

Medical device 100 and/or the means for identifying and/or tracking of medical device 100, respectively, may comprise one or more light sources. For example, medical device 100 and/or the means for identifying and/or tracking may comprise an infrared light source, e.g. an infrared LED light source. Such an infrared LED light source may be adapted to generate one or more spots on a cornea of the patient's eye (e.g. Purkinje reflections) to be used in identifying and/or tracking. For example, the auxiliary camera may be adapted to record one or more of such spots, such that in addition to e.g. x-y coordinates, the mentioned three rotational degrees of freedom can be determined. Additionally or alternatively, medical device 100 and/or the means for identifying and/or tracking may comprise a laser light source adapted to illuminate the eye, such as a blue laser. The main camera 150 may be adapted to record the blue laser light returning from the eye, such that e.g. images showing one or more layers of the cornea can be recorded. Reference is made to European patent application 18711228.9 for details of a technique that can be used for that matter. For example, features such as the thickness profile of the cornea and/or individual layers of the cornea may thus be automatically identified.

Medical device 100 may further comprise means for adjusting a position of a target structure of the patient, i.e., the eye to be examined, relative to at least a portion of medical device 100, e.g., relative to main camera 150 and the auxiliary camera. Such means for adjusting may be adapted to move the chin rest(s) and/or forehead rest(s) of the medical device 100 to effect the relative movement, and the main camera 150 and the auxiliary camera may not move (apart from the mentioned rotation of main camera 150). The movement may be laterally (e.g. in x-y direction) and/or frontally (e.g. in z-direction) relative to the main camera 150, as illustrated by arrows 160a, 160b, 160c in Fig. 1. The means for adjusting a position of a target structure of the patient may comprise at least one motor. In other examples, main camera 150, and/or the axis around which it is adapted to rotate, respectively, may be movable. Thereby, the position of main camera 150, and/or the axis around which it is adapted to rotate, respectively, may be adjusted relative to, e.g., chin rests 110a, 110b and forehead rests 120a, 120b and therefore also relative to the patient's eye to be examined.

The means for adjusting of medical device 100 may be adapted to adjust the relative position of the eye to be examined based at least in part on feedback provided from the means for identifying and/or tracking of medical device 100 as described above.

Medical device 100 may comprise a user interface 190, specifically a (touch)screen. As illustrated in inset 191, user interface 190 may be adapted to provide feedback and/or orders to the patient. For example, as shown in screen 191a, user interface 190 may inform the patient that it is in a "Self Acquisition Mode", i.e., a first mode dedicated for automatically acquiring an exam for medical purposes as described above. Notably, medical device 100 may comprise means for switching medical device 100 into this mode. These means for switching may be operated by trained personnel, in particular medical personnel such as physicians, technicians and/or nurses. Next to this first mode, medical device 100 may also comprise at least one second mode in which at least one functionality of the medical device that is disabled in the first mode is enabled. For example, in the first mode, medical device 100 may not accept any user input, or it may only accept user input for starting and/or stopping exam acquisition as described above, but no other input.

Once exam acquisition has started, user interface 190 may inform the patient that "Self Acquisition Started" and instruct him/her to "Follow the instructions" as illustrated in screen 191b. For example, the patient may be instructed to position his/her chin on one of chin rest 110a and chin rest 110b, depending on which eye of the patient is currently to be examined.

Once exam acquisition has finished successfully, user interface 190 may inform the patient that "Acquisition Succeeded" as illustrated in screen 1910. Such information may be accompanied by a - possibly color coded - smiley face. That is, smiley face shown in screen 1910 is green, evoking positive connotations. Conversely, if exam acquisition did not finish successfully, user interface 190 may inform the patient that "Acquisition Failed" or the like (e.g. using a red color).

Medical device 100 may further comprise means for validating automatic exam acquisition based on comparing a plurality of results obtained from acquiring the exam of the patient a plurality of times. Then, only if automatic exam acquisition is validated, medical device 100 may inform the patient that "Acquisition Succeeded" as illustrated in screen 1910 or that "Acquisition Failed" or the like.

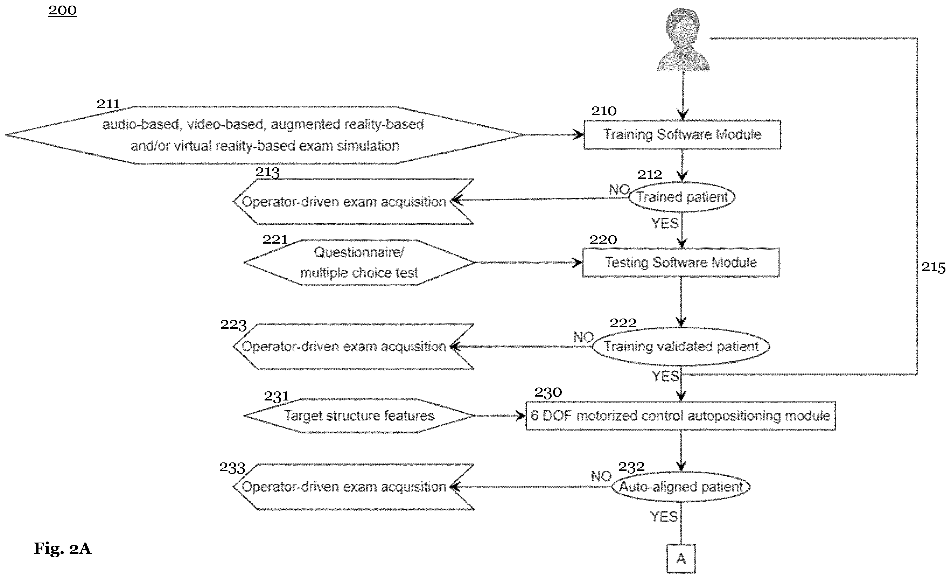

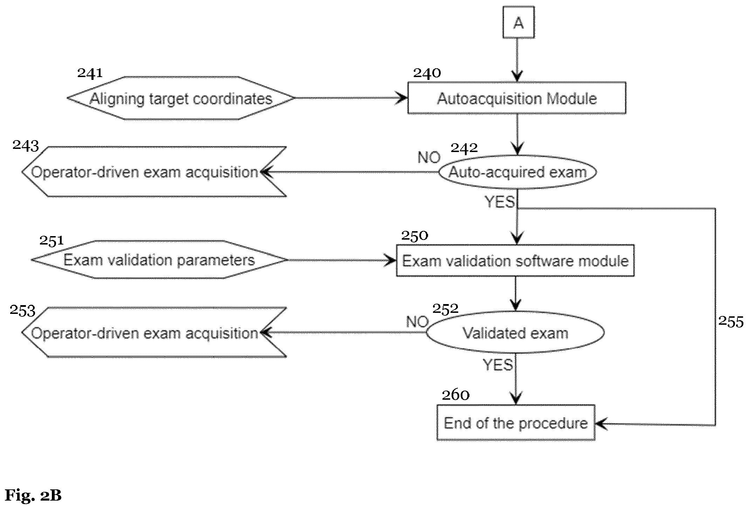

Figs. 2A and 2B show a flowchart 200 illustrating operation of an exemplary medical device for acquiring an exam of a patient for medical purposes according to some aspects of the present invention. In particular, flowchart 200 illustrates steps that may need to be taken for and/or during automatic exam acquisition as already described above in detail. The transition point between the two portions of flowchart 200 shown in Figs. 2A and 2B, respectively, is identified by the boxed letter "A".

Automatic exam acquisition may first present a patient with a training software module 210. Training software module 210 may be comprised in a computer program for authorizing a patient to use a medical device to automatically acquire an exam of the patient for medical purposes as described above. Training software module 210 is based at least in part on an audio-based, video-based, augmented reality-based and/or virtual-reality based exam simulation 211 as described above. Using training software module 210, the patient may be trained to use the medical device for automatic exam acquisition. If the patient has not been (successfully) trained in step 212, exam acquisition is performed by an operator of the medical device, such as trained personnel, in particular medical personnel such as physicians, technicians and/or nurses (operator-driven exam acquisition 213).

If the patient has been (successfully) trained in step 212, he/she may be presented with testing software module 220. Testing software module 220 may be comprised in a computer program for authorizing a patient to use a medical device to automatically acquire an exam of the patient for medical purposes as described above. Testing software module 220 may be based at least in part on a questionnaire and/or a multiple-choice test 221 as described above. Using testing software module 220, the patient may be tested as to whether he/she knows how to use the medical device for automatic exam acquisition. If the patient has not been (successfully) tested in step 222, exam acquisition is performed by the operator (operator-driven exam acquisition 223). If the patient has been (successfully) tested in step 222, he/she may proceed with automatic exam acquisition.

That is, using training software module 210 and testing software module 220, the patient may be authorized to use the medical device for automatically acquiring the exam. This may comprise issuing an authorization to the patient, e.g., a computer-processable authorization to use the medical device to automatically acquire the exam of the patient for medical purposes. Such an authorization may comprise a code, preferably a barcode or a QR code, as described above.

In some examples, the patient may not need to be authorized to use the medical device for automatically acquiring the exam, e.g., he/she may not need to be presented with training software module 210 and testing software module 220. Rather the patient may directly proceed with automatic exam acquisition, as illustrated by loop 215 in flowchart 200, and training software module 210 and testing software module 220 may not be needed. For example, if the exam to be acquired is a very simple one, or one that does not entail any (serious) risks, the patient may be allowed to skip and/or bypass training and/or testing, i.e., he/she may skip and/or bypass authorization. Whether the patient needs to be authorized or not, i.e., whether training software module 210 and testing software module 220 are needed and/or whether he/she needs to be presented with training software module 210 and testing software module 220 prior to automatically acquiring the exam using the medical device, may additionally or alternatively depend on regulations governing the medical sector, e.g., in a given country.

To proceed with automatic exam acquisition, the patient may then need to place himself/herself in front of the medical device. A 6 degree of freedom motorized control autopositioning module 230 (comprising, e.g., means for identifying and/or tracking a target structure of the patient as described above) may identify and/or track a target structure of the patient as described above. Additionally or alternatively, 6 degree of freedom motorized control autopositioning module 230 (comprising, e.g., means for adjusting a position of a target structure of the patient relative to at least a portion of the medical device as described above) may be used to adjust a position of the target structure of the patient relative to at least a portion of the medical device. 6 degree of freedom motorized control autopositioning module 230 may be based at least in part on target structure features 231. Target structure features 231 may, e.g., be stored and/or embedded in the authorization that may be issued to the patient as described above. For example, a pupil may be such a target structure. Additionally, also further features e.g. of an eye may be used. Using 6 degree of freedom motorized control autopositioning module 230, the patient may be auto-aligned. In other words, the target structure to be examined may be identified and tracking may be operational. If the patient has not been (successfully) auto-aligned in step 232, exam acquisition is performed by the operator (operator-driven exam acquisition 233).

If the patient has been (successfully) auto-aligned in step 232, he/she may be allowed to use autoacquisition module 240 which is based at least in part on aligning target coordinates 241 (note the transition between the two portions of flowchart 200 shown in Figs. 2A and 2B, respectively, as identified by the boxed letter "A"). For example, the 6 degree of freedom motorized control autopositioning module 230 (comprising, e.g., means for identifying and/or tracking a target structure of the patient as described above) may ensure that - throughout the autoacquisition - the target structure remains aligned. Using autoacquisition module 240, the (actual) exam is automatically acquired as described in detail above. If the exam is not acquired (successfully) in step 242, exam acquisition is performed by the operator (operator-driven exam acquisition 243).

If the exam is acquired (successfully) in step 242, exam validation software module 250 may be employed (comprising, e.g., means for validating automatic exam acquisition based on comparing a plurality of results obtained from acquiring the exam of the patient a plurality of times). Exam validation software module 250 may be based at least in part on exam validation parameters 251. Exam validation parameters 251may comprise predefined margins and/or standards. For exam validation, it may be determined whether one or more exam results remain within these predefined margins and/or standards. If the exam is not validated (successfully) in step 252, exam acquisition is performed by the operator (operator-driven exam acquisition 253). If the exam is validated (successfully) in step 252, end of procedure 260 is reached.

In some examples, no exam validation software module such as exam validation software module 250 may be employed. Then, if the exam is acquired (successfully) in step 242, exam validation software module 250 may be skipped and/or bypassed, and end of procedure 260 may be reached immediately, as illustrated by loop 255 in flowchart 200.

## Claims

1. A system comprising:
a computer program for authorizing a patient to use a medical device (100) to automatically acquire an exam of the patient for medical purposes, configured to be executed on a first device and comprising code for:
determining one or more criteria based at least in part on one or more properties of the medical device (100);
verifying whether the patient meets at least one of the criteria; and
issuing a code to the patient;
wherein the computer program comprises code for, prior to authorizing the patient, providing the patient with a training regarding the medical device (100), in particular wherein the training is at least partly audio-based, video-based, augmented reality-based and/or virtual reality-based; and
a medical device (100) for acquiring an exam of a patient for medical purposes,
wherein the medical device comprises means for automatically acquiring the exam and means for determining whether the patient is authorized to use the medical device for automatically acquiring the exam;
wherein the means for determining whether the patient is authorized are adapted to determine whether the patient is authorized based at least in part on the code, preferably a barcode or a quick response, QR, code, issued to the patient upon completion of the training;
wherein the medical device further comprises means for acquiring the code, wherein the medical device comprises means for validating automatic exam acquisition based on comparing a plurality of results obtained from acquiring the exam of the patient a plurality of times; and
wherein the medical device is distinct from the first device.

2. The system according to claim 1, wherein the medical device (100) is not a wearable device.

3. The system according to claim 1 or 2, wherein the medical device (100) is an ophthalmic medical device.

4. The system according to any of the preceding claims, wherein the medical device (100) comprises means for switching the medical device (100) into a first mode dedicated for automatically acquiring an exam for medical purposes.

5. The system according to claim 4, wherein the medical device (100) comprises means for switching the medical device (100) into at least one second mode in which at least one functionality of the medical device that is disabled in the first mode is enabled.

6. The system according to any of the preceding claims, wherein the medical device (100) comprises means for identifying and/or tracking a target structure of the patient, in particular wherein the means for identifying and/or tracking comprise one or more algorithms for matching the target structure and a reference structure.

7. The system according to any of the preceding claims, wherein the medical device (100) comprises means for adjusting a position of a target structure of the patient relative to at least a portion of the medical device; in particular wherein the means for adjusting comprise at least one motor.

8. The system according to claim 7 when referred back to claim 6, wherein the means for adjusting are adapted to adjust the position of the target structure based at least in part on feedback provided from the means for identifying and/or tracking.

9. The system according to any of the preceding claims, wherein the medical device (100) comprises a user interface (190), preferably comprising one or more of the following: a screen, a touchscreen, an audio output; in particular wherein the user interface is adapted to provide feedback and/or orders to the patient.

## Patentansprüche

1. System, umfassend:
ein Computerprogramm zum Autorisieren eines Patienten, eine medizinische Vorrichtung (100) zu verwenden, um automatisch eine Prüfung des Patienten für medizinische Zwecke zu erwerben, das konfiguriert ist, um auf einer ersten Vorrichtung ausgeführt zu werden, und Code umfasst zum:
Bestimmen eines oder mehrerer Kriterien basierend zumindest teilweise auf einer oder mehreren Eigenschaften der medizinischen Vorrichtung (100);
Verifizieren, ob der Patient mindestens eines der Kriterien erfüllt; und
Ausgeben eines Codes an den Patienten;
wobei das Computerprogramm Code umfasst, um vor dem Autorisieren des Patienten dem Patienten ein Training bezüglich der medizinischen Vorrichtung (100) bereitzustellen, insbesondere wobei das Training zumindest teilweise audiobasiert, videobasiert, Augmented-Reality-basiert und/oder Virtual-Reality-basiert ist; und
eine medizinische Vorrichtung (100) zum Erwerben einer Prüfung eines Patienten für medizinische Zwecke,
wobei die medizinische Vorrichtung Mittel zum automatischen Erwerben der Prüfung und Mittel zum Bestimmen, ob der Patient autorisiert ist, die medizinische Vorrichtung zum automatischen Erwerben der Prüfung zu verwenden, umfasst;
wobei die Mittel zum Bestimmen, ob der Patient autorisiert ist, angepasst sind, um zu bestimmen, ob der Patient autorisiert ist, basierend zumindest teilweise auf dem Code, vorzugsweise einem Barcode oder einem Quick-Response-Code, QR-Code, der an den Patienten nach Abschluss des Trainings ausgegeben wird;
wobei die medizinische Vorrichtung ferner Mittel zum Erwerben des Codes umfasst, wobei die medizinische Vorrichtung Mittel zum Validieren der automatischen Prüfungserwerbung basierend auf dem Vergleichen einer Vielzahl von Ergebnissen, die aus dem mehrmaligen Erwerben der Prüfung des Patienten erhalten werden, umfasst; und
wobei sich die medizinische Vorrichtung von der ersten Vorrichtung unterscheidet.

2. System nach Anspruch 1, wobei die medizinische Vorrichtung (100) keine tragbare Vorrichtung ist.

3. System nach Anspruch 1 oder 2, wobei die medizinische Vorrichtung (100) eine ophthalmische medizinische Vorrichtung ist.

4. System nach einem der vorhergehenden Ansprüche, wobei die medizinische Vorrichtung (100) Mittel zum Umschalten der medizinischen Vorrichtung (100) in einen ersten Modus, der zum automatischen Erwerben einer Prüfung für medizinische Zwecke bestimmt ist, umfasst.

5. System nach Anspruch 4, wobei die medizinische Vorrichtung (100) Mittel zum Umschalten der medizinischen Vorrichtung (100) in mindestens einen zweiten Modus, in dem mindestens eine Funktionalität der medizinischen Vorrichtung, die im ersten Modus deaktiviert ist, aktiviert ist, umfasst.

6. System nach einem der vorhergehenden Ansprüche, wobei die medizinische Vorrichtung (100) Mittel zum Identifizieren und/oder Verfolgen einer Zielstruktur des Patienten umfasst, insbesondere wobei die Mittel zum Identifizieren und/oder Verfolgen einen oder mehrere Algorithmen zum Abgleichen der Zielstruktur und einer Referenzstruktur umfassen.

7. System nach einem der vorhergehenden Ansprüche, wobei die medizinische Vorrichtung (100) Mittel zum Einstellen einer Position einer Zielstruktur des Patienten relativ zu mindestens einem Abschnitt der medizinischen Vorrichtung umfasst; insbesondere wobei die Mittel zum Einstellen mindestens einen Motor umfassen.

8. System nach Anspruch 7, wenn auf Anspruch 6 rückbezogen, wobei die Mittel zum Einstellen angepasst sind, um die Position der Zielstruktur basierend mindestens teilweise auf Rückkopplung einzustellen, die von den Mitteln zum Identifizieren und/oder Verfolgen bereitgestellt wird.

9. System nach einem der vorhergehenden Ansprüche, wobei die medizinische Vorrichtung (100) eine Benutzerschnittstelle (190) umfasst, die vorzugsweise eines oder mehrere der Folgenden umfasst: einen Bildschirm, einen Touchscreen, einen Audioausgang; insbesondere wobei die Benutzerschnittstelle angepasst ist, um Rückkopplung und/oder Befehle an den Patienten bereitzustellen.

## Revendications

1. Un système comprenant :
un programme informatique pour autoriser un patient à utiliser un dispositif médical (100) pour procéder automatiquement à un examen du patient à des fins médicales, configuré pour être exécuté sur un premier dispositif et comprenant du code pour :
déterminer un ou plusieurs critères basés au moins en partie sur une ou plusieurs propriétés du dispositif médical ;
vérifier si le patient répond ou non à au moins l'un des critères ; et
délivrer un code au patient ;
dans lequel le programme informatique comprend du code pour, avant d'autoriser le patient, délivrer au patient une formation relative au dispositif médical (100), en particulier dans lequel la formation est au moins partiellement basée sur de l'audio, basée sur de la vidéo, basée sur de la réalité augmentée et/ou basée sur de la réalité virtuelle ; et
un dispositif médical (100) pour procéder à un examen d'un patient à des fins médicales,
dans lequel le dispositif médical comprend des moyens pour procéder automatiquement à l'examen et des moyens pour déterminer si le patient est ou non autorisé à utiliser le dispositif médical pour procéder automatiquement à l'examen ;
dans lequel les moyens pour déterminer si le patient est ou non autorisé sont aptes à déterminer si le patient est ou non autorisé au moins en partie sur la base du code, de préférence d'un code à barres ou d'un code à réponse rapide, QR, délivré au patient à l'achèvement de la formation ;
dans lequel le dispositif médical comprend en outre des moyens pour acquérir le code, le dispositif médical comprenant des moyens pour valider une acquisition automatique de l'examen sur la base d'une comparaison d'une pluralité de résultats obtenus d'après l'examen du patient qui a été procédé une pluralité de fois ; et
dans lequel le dispositif médical est distinct du premier dispositif.

2. Le système selon la revendication 1, dans lequel le dispositif médical (100) n'est pas un dispositif à porter sur soi.

3. Le système selon la revendication 1 ou 2, dans lequel le dispositif médical (100) est un dispositif médical ophtalmique.

4. Le système selon l'une des revendications précédentes, dans lequel le dispositif médical (100) comprend des moyens pour commuter le dispositif médical (100) en un premier mode dédié destiné à procéder automatiquement à l'examen à des fins médicales.

5. Le système selon la revendication 4, dans lequel le dispositif médical (100) comprend des moyens pour commuter le dispositif médical (100) dans au moins un second mode dans lequel est activée au moins une fonctionnalité du dispositif médical qui est désactivée dans le premier mode.

6. Le système selon l'une des revendications précédentes, dans lequel le dispositif médical (100) comprend des moyens pour identifier et/ou suivre une structure cible du patient, en particulier dans lequel les moyens pour identifier et/ou pour suivre comprennent un ou plusieurs algorithmes pour faire concorder la structure cible avec une structure de référence.

7. Le système selon l'une des revendications précédentes, dans lequel le dispositif médical (100) comprend des moyens pour ajuster une position d'une structure cible du patient par rapport à au moins une partie du dispositif médical, en particulier dans lequel les moyens pour ajuster comprennent au moins un moteur.

8. Le système selon la revendication 7 prise en dépendance de la revendication 6, dans lequel les moyens pour ajuster sont aptes à ajuster la position de la structure cible au moins en partie sur la base d'une réponse produite par les moyens pour identifier et/ou pour suivre.

9. Le système selon l'une des revendications précédentes, dans lequel le dispositif médical (100) comprend une interface utilisateur (190), comprenant de préférence un ou plusieurs d'entre : un écran, un écran tactile, une entrée audio ; en particulier dans lequel l'interface utilisateur est apte à produire un retour et/ou des ordres destinés au patient.
